# EUROPÄISCHE PATENTSCHRIFT

(11) **EP 4 031 210 B1**
(45) Veröffentlichungstag und Bekanntmachung des Hinweises auf die Patenterteilung: **17.12.2025**
(21) Anmeldenummer: 20786241.8
(22) Anmeldetag: 15.09.2020
(51) Int. Cl.: A61M 5/178, B65B 3/00, B65B 7/28, A61M 5/31

(54) **VORRICHTUNG UND VERFAHREN ZUM VERSCHLIESSEN VON SPRITZENKÖRPERN**
DEVICE AND METHOD FOR CLOSING SYRINGE BODIES
DISPOSITIF ET PROCÉDÉ DE FERMETURE DE CORPS DE SERINGUE

(30) Priorität: 16.09.2019 DE 102019214071
(43) Veröffentlichungstag der Anmeldung: 27.07.2022
(73) Patentinhaber: Brugger, Stefan, 88483 Burgrieden/Rot (DE); Klotz, Margit, 88471 Laupheim (DE)
(72) Erfinder: Brugger, Stefan, 88483 Burgrieden/Rot (DE); Klotz, Margit, 88471 Laupheim (DE)
(74) Vertreter: Sawodny, Michael-Wolfgang
(86) Internationale Anmeldenummer: PCT/EP2020/075711
(87) Internationale Veröffentlichungsnummer: WO 2021/052931

(56) Entgegenhaltungen:
- KR-B1- 101 617 853
- US-A- 1 990 301
- US-A- 5 519 984
- US-A1- 2006 168 916

## Beschreibung

Die vorliegende Erfindung betrifft eine Vorrichtung zum manuellen Einführen eines Kolbenstopfens aus Elastomer, der einen Teil eines Kolbens bildet, in einen Spritzenzylinder,

Ein Verfahren und eine Vorrichtung um einen Kolbenstopfen in einen Spritzenzylinder einzuführen, ist aus der US 2006/168916 A1 bekannt geworden. Bei dem in der US 2006/0168916 A1 beschriebenen Verfahren und der Vorrichtung wird ein Vakuum zum Einsetzen des Stopfens in eine Spritze eingesetzt.

Das Einsetzen eines Stopfens in eine Spritze hat auch die US 5,519,984 A zum Gegenstand, ebenso wie die KR 101 617 853 B1.

Die US 1990301 A beschreibt eine Vorrichtung zum Verschließen von Flaschen bspw. mittels Korken.

Die bisher verwendeten Verfahren zum Verschließen von Spritzen mit Kolbenstopfen sind entweder sehr aufwendig (Einsatz von Füllmaschinen) oder nicht steril bzw. repräsentativ (Manipulation des Kolbenstopfens während des Setzens zur Vermeidung eines Überdrucks). In einer frühen Phase der Entwicklung oder während eines Life Cycle Managements eines flüssigen Arzneimittels oder Medizinprodukts, dessen Darreichungsform eine Spritze sein soll, gibt es grundsätzlich zwei Gründe, welche das Verschließen von Spritzen mit Kolbenstopfen außerhalb einer Routine-Produktion im Großmaßstab erforderlich machen:
- Es besteht die Notwendigkeit, die Materialkompatibilität bezüglich der Wechselwirkung zwischen Lösung und Packmittel über einen längeren Zeitraum (möglichst entsprechend der angestrebten Haltbarkeit des Arzneimittels) zu untersuchen, zu analysieren und gegebenenfalls die Formulierung oder die Packmittel anzupassen. Hierbei ist es wesentlich, dass die Art und Weise der Herstellung möglichst nahe einer späteren industriellen Herstellung mit einer Füllmaschine entspricht. Bereits kleinere Abweichungen können die Analysenergebnisse so massiv beeinflussen, dass eine Aussage über die Kompatibilität nicht mehr belastbar möglich ist. Dies kann in späteren Phasen der Entwicklung eines Arzneimittels zu erheblichen Problemen führen (Verringerung der Haltbarkeit) oder gar zu einer Neu-Entwicklung der Formulierung / der Packmittel, was einen erheblichen Aufwand an Zeit und Kosten verursacht;
- Toxikologische Studien sowie in-vivo-Tests sind oft in frühen Phasen der Arzneimittelentwicklung gefordert. Für diese Tests müssen die angestrebten sterilen Arzneimittel auch eine tatsächlich Sterilität aufweisen. Da diese durch Manipulation der Kolbenstopfen beim herkömmlichen manuellen Stopfensetzen nicht gewährleistet ist, kann diese Art der Herstellung nicht verwendet werden. Ebenso schwierig ist die Abfüllung auf einer Füllmaschine, da hier GMP-Regeln nicht erlauben, Substanzen mit nicht bekannten toxikologischen Eigenschaften in die Räumlichkeiten einzubringen (Vermeidung Cross-Kontamination, Reinigungs-Validierung nicht möglich). Zudem ist oft die zur Verfügung stehende Batchgröße für ein automatisiertes Stopfensetzen auf einer Füllmaschine nicht ausreichend (Totvolumina).

Es bestand daher eine Notwendigkeit, eine Vorrichtung bzw. ein Verfahren bereitzustellen, welche(s) das oben erwähnte Befüllen von Spritzen auch in Kleinstserien ermöglicht.

In einem ersten Aspekt der vorliegenden Erfindung wird diese Aufgabe durch eine Vorrichtung zum manuellen Einführen eines Kolbenstopfens aus Elastomer, der einen Teil eines Kolbens bildet, in einen Spritzenzylinder gelöst, umfassend: eine Aufnahme, die dazu eingerichtet ist, den Spritzenzylinder zu haltern; ein röhrenförmiges Setzrohr, das dazu eingerichtet ist, in seinem Inneren den Kolbenstopfen aufzunehmen; eine Haltevorrichtung, die dazu eingerichtet ist, das Setzrohr relativ zu der Aufnahme zu haltern; und eine Verlagerungseinrichtung, die dazu eingerichtet ist, den Kolbenstopfen relativ zu dem Setzrohr zu verlagern; wobei die Haltevorrichtung einen Betätigungsabschnitt umfasst, der dazu eingerichtet ist, manuell betätigt zu werden, um das Setzrohr relativ zu dem Kolbenstopfen zu verlagern.

Die erfindungsgemäße Vorrichtung ermöglicht es, eine Serie an befüllten Spritzen (und anderen spritzenähnlichen zylindrischen Röhren wie z.B.

Karpulen, Doppelkammerkarpulen und Doppelkammerspritzen) ab der Menge von 1 herzustellen. Zusätzlich kann mit der erfindungsgemäßen Vorrichtung eine jeweilige Spritze aseptisch einwandfrei und für verschiedene Einsatzzwecke befüllt werden. Dies führt zu einer deutlichen Reduktion an Kosten und Zeit für die bisher beschriebenen Einsatzgebiete.

Insbesondere erlaubt die erfindungsgemäße Vorrichtung, eine Befüllungsabfolge von vollautomatisierten Befüllungsanlagen nachzuahmen, so dass ein späterer Umstieg von der Kleinserie zu der tatsächlichen Serie der Arzneimittelproduktion ohne größere Umstellungen möglich ist. Dabei können auch für die erfindungsgemäße Vorrichtung Bauteile einer Füllmaschine direkt entnommen werden, um absolut gleiche Bedingungen für die Eigenschaften der hergestellten Spritze zu erreichen. Beispielsweise kommen hier das Setzrohr und der Stößel (siehe unten) in Frage.

Durch die erfindungsgemäße Vorrichtung kann ein jeweiliger Kolbenstopfen gesetzt werden, ohne dass es zur Bildung eines Überdrucks im Spritzenzylinder kommt, wodurch beispielsweise ungewollt Produkt ausgetrieben werden könnte. Im Zusammenhang mit der vorliegenden Erfindung wird deshalb auch von einem "druckfreien" Stopfensetzen gesprochen.

Die Kolbenstopfen können somit absolut präzise positioniert werden, so dass ein gewünschter und erforderlicher "Headspace" sowie eine Gleichförmigkeit über einen gesamten Batch erhalten werden kann.

Dabei können zur Setz-Begasung Schutzgase oder andere Gase angeschlossen werden. Ganz generell kann die erfindungsgemäße Vorrichtung entlang der gesamten Entwicklungskette (Formulierungsentwicklung, Stabilitätsstudien, Toxizitäts-Studien, klinische Studien, Packmittelwechsel bis Life Cycle Management) auch in einer aseptischen Umgebung eingebracht und betrieben werden (von Labor bis Isolator).

Vorteilhafterweise sind zumindest sämtliche Bauteile, welche mit Teilen der zu befüllenden Spritze und/oder dem Arzneimittel in Kontakt kommen, insbesondere alle Bauteile, der erfindungsgemäßen Vorrichtung autoklavierbar.

Ferner kann es vorteilhaft sein, dass zumindest die Aufnahme und/oder das Setzrohr derart ausgebildet sind/ist, dass sie/es durch ein Pendant für eine Spritze eines anderen Formats ausgetauscht werden können/kann. Auf diese Weise kann zum Beispiel erreicht werden, dass Spritzen etwa der Formate 1 ml, 3 ml oder 10 ml befüllt werden können.

Bei der erfindungsgemäßen Vorrichtung kann beispielsweise zuerst ein bereits befüllter Spritzenzylinder in der Aufnahme angeordnet werden, dann wird der Spritzenzylinder relativ zu dem Setzrohr derart verlagert, dass das dem Arzneimittel zugeordnete Ende des Setzrohrs zu dem Arzneimittel einen vorbestimmten Abstand einnimmt. Nun kann ein Kolbenstopfen durch das Setzrohr verlagert werden, wobei in dem Setzrohr zwischen Kolbenstopfen und Arzneimittel angeordnetes Gas bei einer Verlagerung des Kolbenstopfens in dem Setzrohr ausgestoßen werden kann und zwischen Setzrohr und Spritzenzylinder aus dem Spritzenzylinder austreten kann, ohne dass es zu einer Bildung eines Überdrucks in dem Spritzenzylinder kommen würde. Ist der Kolbenstopfen an dem dem Arzneimittel benachbarten Ende des Setzrohrs angeordnet, so betätigt ein Bediener der erfindungsgemäßen Vorrichtung den Betätigungsabschnitt, wodurch das Setzrohr relativ zu dem Kolbenstopfen und von dem Arzneimittel weg verlagert wird. Dabei expandiert sich der Kolbenstopfen derart, dass er mit dem Spritzenzylinder in einer abdichtenden Weise zusammenwirkt. Die fertige Spritze kann nun aus der erfindungsgemäßen Vorrichtung entnommen werden.

In einer Weiterbildung der vorliegenden Erfindung kann der Betätigungsabschnitt der Haltevorrichtung eine Wippeinrichtung umfassen, die dazu eingerichtet ist, eine in einer ersten Richtung wirkende manuelle Betätigungskraft, die an einem ersten Ende der Wippeinrichtung eingegeben worden ist, in eine in einer zweiten Richtung wirkende Hebelkraft an einem zweiten Ende der Wippeinrichtung, das dem ersten Ende entgegengesetzt angeordnet ist, zu übertragen, wobei die erste Richtung der zweiten Richtung im Wesentlichen entgegengesetzt sein kann. Eine derartige Einrichtung kann es ermöglichen, eine geringe Betätigungskraft an der einen Seite in eine vergleichsweise größere Verlagerungskraft auf der anderen Seite umzuwandeln, so dass eine Betätigung des Betätigungsabschnitts vereinfacht werden kann.

Dabei kann die Wippeinrichtung als längliches plattenartiges Element ausgestaltet sein. Dies kann eine besonders einfache und kostengünstige Ausbildung der Wippeinrichtung darstellen.

Ferner kann dabei das plattenartige Element ein erstes Ende, das dazu eingerichtet ist, mit einem Finger betätigt zu werden, und ein zweites Ende, das dem ersten Ende im Wesentlichen entgegengesetzt angeordnet ist und welches das Setzrohr zumindest teilweise umschließt, aufweisen. Das plattenartige Element kann daher an der erfindungsgemäßen Vorrichtung insbesondere derart angeordnet sein, dass es für einen Benutzer gut zugänglich ist, damit dieser mit seinem Finger auf das erste Ende des plattenartigen Elements einwirken kann.

Vorteilhafterweise kann die Vorrichtung ferner mindestens ein Stützelement umfassen, mit dem die Haltevorrichtung und/oder die Verlagerungseinrichtung verbunden sind/ist.

Auch kann die Vorrichtung ferner eine Grundplatte umfassen, die vorteilhafterweise mit dem mindestens einen Stützelement verbunden sein kann. Das wenigstens eine Stützelement und die Grundplatte können so zusammen ein Gestell bilden, an welchem die oben beschriebenen Teile der erfindungsgemäßen Vorrichtung gehaltert sein können.

Insbesondere kann die Aufnahme an der Grundplatte angebracht sein. Dadurch kann erreicht werden, dass die Aufnahme gegenüber der Grundplatte abgestützt ist und ein zum Beispiel horizontales Spiel der Aufnahme gegenüber der Grundplatte reduziert oder sogar vermieden werden kann.

Die Aufnahme kann relativ zu der Haltevorrichtung und/oder der Verlagerungseinrichtung justierbar sein, vorteilhafterweise über mindestens eine Schraubverbindung. Hierdurch kann der Spritzenzylinder in der Vorrichtung verlagert werden. Ferner kann so die erfindungsgemäße Vorrichtung an das Befüllen von Spritzen mit zueinander unterschiedlichen Füllständen bzw. unterschiedlichen Spritzenformaten angepasst werden.

Vorteilhafterweise können die Haltevorrichtung und/oder die Verlagerungseinrichtung und/oder die Aufnahme relativ zueinander justierbar sein, vorteilhafterweise über jeweils mindestens eine Schraubverbindung. Auf diese Weise kann eine maximale Flexibilität beim Herstellen der Spritzen und/oder beim Anpassen der erfindungsgemäßen Vorrichtung an unterschiedliche Spritzenformate und/oder Füllstände erreicht werden.

Das Setzrohr kann ferner dazu eingerichtet sein, derart relativ zu der Aufnahme verlagerbar zu sein, dass es in einer vorbestimmten Position seines Verlagerungsweges zumindest teilweise in einen Innenraum eines an der Aufnahme angeordneten Spritzenzylinders hineinragt. Somit kann das Setzrohr mit einem darin angeordneten Kolbenstopfen so weit in den Spritzenzylinder hinein verlagert werden, bis das Setzrohr in der Nähe eines in dem Spritzenzylinder angeordneten Arzneimittels ist.

Insbesondere kann das Setzrohr an einem Ende einen sich radial nach außen erstreckenden Kragen aufweisen. Das Setzrohr kann so in eine entsprechende Halterung beispielsweise von oben eingesetzt werden und aufgrund des Kragens und der Schwerkraft gehalten werden. Dies kann eine einfache Austauschbarkeit des Setzrohrs an der erfindungsgemäßen Vorrichtung ermöglichen.

Ferner kann die zum Haltern des Spritzenzylinders eingerichtete Aufnahme mindestens zwei Teile umfassen.

Dabei kann die Aufnahme eine erste Zentrieraufnahme, die dazu eingerichtet ist, eine zweite Zentrieraufnahme zu haltern, und die zweite Zentrieraufnahme, die dazu eingerichtet ist, den Spritzenzylinder zu haltern, umfassen, wobei die zweite Zentrieraufnahme in die erste Zentrieraufnahme zumindest teilweise eintaucht.

Die erste Zentrieraufnahme kann einen Tiefenanschlag aufweisen, der dazu eingerichtet ist, den Verlagerungsweg der zweiten Zentrieraufnahme zu begrenzen. Mit anderen Worten, der Verlagerungsweg der zweiten Zentrieraufnahme kann durch die erste Zentrieraufnahme definiert werden.

Die zweite Zentrieraufnahme kann an ihrem der ersten Zentrieraufnahme im Wesentlichen entgegengesetzten Ende eine Halterung für einen Spritzenzylinder aufweisen, die vorteilhafterweise hohlzylinderförmig oder klammerartig ausgestaltet ist. In diese Halterung der zweiten Zentrieraufnahme kann dann ein jeweiliger Spritzenzylinder entweder von oben eingesetzt werden oder seitlich, in einer beispielsweise horizontalen Richtung, eingerastet werden, wenn die Aufnahme zum Beispiel als elastische Klammer ausgebildet ist. Dabei kann das Arzneimittel zu diesem Zeitpunkt bereits im Spritzenzylinder vorhanden sein oder erst nach dessen Justierung in der Halterung der zweiten Zentrieraufnahme eingefüllt werden.

Ferner kann die zum Haltern des Setzrohrs eingerichtete Haltevorrichtung das Setzrohr zumindest teilweise umschließen. Ein teilweises Umschließen kann insbesondere derart sein, dass das Setzrohr lediglich entlang seiner Längserstreckungsrichtung verlagerbar aufgenommen ist.

Dabei kann die Haltevorrichtung hohlzylinderförmig mit einer bevorzugt zur Mittelachse der Haltevorrichtung konzentrischen Bohrung zur Aufnahme des Setzrohrs ausgestaltet sein.

In einer Weiterbildung der vorliegenden Erfindung kann die zum Verlagern des Kolbenstopfens relativ zum Setzrohr eingerichtete Verlagerungseinrichtung eine Stößelführung, die dazu eingerichtet ist, einen Stößel relativ zu dem Setzrohr auszurichten, und den Stößel, der dazu eingerichtet ist, den Kolbenstopfen relativ zum Setzrohr zu verlagern, umfassen. Der Stößel kann hier zum Beispiel als eine Stange ausgebildet sein, welche insbesondere parallel, vorteilhafterweise koaxial, zu einer Längsmittelachse des Spritzenzylinders verlagerbar ist.

Dabei kann die Verlagerungseinrichtung ferner eine Stößelführungsaufnahme umfassen, die dazu eingerichtet ist, die Stößelführung zu haltern. Zum Beispiel kann die Stößelführung als Gleitbuchse ausgebildet sein.

Ferner kann die Stößelführung oder die Stößelführungsaufnahme mit dem mindestens einen Stützelement verbunden sein, wobei die Stößelführung vorteilhafterweise direkt oder indirekt justierbar sein kann, vorteilhafterweise über mindestens eine Schraubverbindung. Auch hier kann eine Einstellbarkeit der Stößelführung erlauben, dass die erfindungsgemäße Vorrichtung an unterschiedliche Spritzenformate angepasst werden kann.

Vorteilhafterweise kann der Stößel einen zylindrischen Abschnitt an dem der Haltevorrichtung zugewandten Ende aufweisen, dessen Außendurchmesser kleiner ist als ein lichter Innendurchmesser des Setzrohrs. Dies kann es dem Stößel ermöglichen, in das Setzrohr einzutauchen, um den Kolbenstopfen dort hindurch zu verlagern. Ein nur geringfügig kleinerer Durchmesser dieses Abschnitts des Stößels im Vergleich zu dem Innendurchmesser des Setzrohrs kann ein ungewolltes Verkippen des Kolbenstopfens im Setzrohr verhindern.

Der Stößel kann ferner an dem der Haltevorrichtung entgegengesetzten Ende einen sich vorteilhafterweise radial nach außen erstreckenden Kopf umfassen, der vorteilhafterweise dazu eingerichtet ist, manuell, vorteilhafterweise mit einem Finger, betätigt zu werden, um den Kolbenstopfen relativ zu dem Setzrohr zu verlagern. Der Kopf des Stößels kann einerseits eine ausreichend große Betätigungsfläche bieten, damit ein Bediener den Stößel per Hand verlagern kann. Andererseits kann der Kopf des Stößels auch als Gewicht dienen, um den Bediener dabei zu unterstützen, die Kraft aufzubringen, welche notwendig ist, um den Kolbenstopfen durch das Setzrohr zu verlagern.

Insbesondere kann der Stößel ferner einen vorteilhafterweise justierbaren Tiefenanschlag umfassen, der dazu eingerichtet ist, das Ende des Verlagerungsweges des Kolbenstopfens in dem Setzrohr durch die Verlagerungseinrichtung festzulegen. Dies kann es ermöglichen, dass ein Bediener, nach erfolgreich justiertem Tiefenanschlag und gleich bleibendem Spritzenformat/Füllstand, den Stößel bis zu der Begrenzung verlagern kann, ohne dabei auf die Position des Stößels relativ zu dem Spritzenzylinder achten zu müssen. Sobald der Stößel seinen maximalen Verlagerungsweg erreicht hat, kann der Bediener den Betätigungsabschnitt, beispielsweise die Wippeinrichtung, betätigen, wodurch der Kolbenstopfen wie oben beschrieben freigegeben wird.

Ferner kann die Vorrichtung eine Sicherungseinrichtung umfassen, die dazu eingerichtet ist, eine unbeabsichtigte Verlagerung des Kolbenstopfens relativ zu dem Setzrohr zu verhindern, wobei die Sicherungseinrichtung vorteilhafterweise schwenkbar mit dem mindestens einen Stützelement verbunden ist, vorteilhafterweise über mindestens eine Schraubverbindung. Somit kann die Schraubverbindung ein Verschwenken der Sicherungseinrichtung in einem befestigten Zustand verhindern und in einem gelösten Zustand erlauben. Die Sicherungseinrichtung kann auch mit einem elastischen Element, wie beispielsweise einer Feder, ausgerüstet sein, so dass sich die Sicherungseinrichtung selbstständig aus einem Freigabezustand, in welcher eine Verlagerung des Stößels erlaubt ist, in einen Sicherungszustand verlagert, in welcher eine Verlagerung des Stößels gehemmt ist.

In einer vorteilhaften Ausführungsform der Vorrichtung kann diese umfassen: eine Grundplatte;
ein Stützelement, das mit der Grundplatte verbunden ist;
eine vorteilhafterweise an der Grundplatte angebrachte Aufnahme umfassend
eine erste Zentrieraufnahme, die dazu eingerichtet ist, eine zweite Zentrieraufnahme zu haltern und die vorteilhafterweise einen Tiefenanschlag aufweist, und
die zweite Zentrieraufnahme, die dazu eingerichtet ist, den Spritzenzylinder zu haltern;
ein röhrenförmiges Setzrohr, das dazu eingerichtet ist, in seinem Inneren den Kolbenstopfen aufzunehmen;
eine vorteilhafterweise an dem Stützelement angebrachte Haltevorrichtung, die dazu eingerichtet ist, das Setzrohr relativ zu der Aufnahme zu haltern und die vorteilhafterweise das Setzrohr zumindest teilweise umschließt, wobei die Haltevorrichtung eine als längliches plattenartiges Element ausgestaltete Wippeinrichtung umfasst, die dazu eingerichtet ist, das Setzrohr relativ zu dem Kolbenstopfen zu verlagern, wobei die Wippeinrichtung vorteilhafterweise ein erstes Ende, das dazu eingerichtet ist, mit einem Finger betätigt zu werden, und ein zweites Ende, das dem ersten Ende im Wesentlichen entgegengesetzt angeordnet ist und welches das Setzrohr zumindest teilweise umschließt, aufweist, und
eine Verlagerungseinrichtung, umfassend
eine Stößelführung, die dazu eingerichtet ist, einen Stößel relativ zu dem Setzrohr auszurichten, wobei die Stößelführung vorteilhafterweise direkt oder indirekt justierbar ist;
den Stößel, der dazu eingerichtet ist, den Kolbenstopfen relativ zum Setzrohr zu verlagern und der vorteilhafterweise einen Tiefenanschlag aufweist, und eine vorteilhafterweise an dem Stützelement angebrachte Stößelführungsaufnahme, die dazu eingerichtet ist, die Stößelführung zu haltern; und gegebenenfalls
eine Sicherungseinrichtung, die dazu eingerichtet ist, eine unbeabsichtigte Verlagerung des Kolbenstopfens relativ zu dem Setzrohr zu verhindern, wobei die Sicherungseinrichtung vorteilhafterweise schwenkbar mit dem mindestens einen Stützelement verbunden ist. In Bezug auf diese spezielle Ausführungsform der vorliegenden Erfindung sei auf die zu den entsprechenden einzelnen Merkmalen angeführten Beschreibungen verwiesen.

In einem zweiten Aspekt betrifft die vorliegende Erfindung ein Verfahren zum manuellen flüssigkeitsdichten Einführen eines Kolbenstopfens aus Elastomer, der einen Teil eines Kolbens bildet, in einen Spritzenzylinder, umfassend die Schritte:
(i) Einführen des Kolbenstopfens in ein röhrenförmiges Setzrohr;
(ii) Einführen des Spritzenzylinders in eine dazu eingerichtete Aufnahme ;
(iii) Verlagern des Kolbenstopfens innerhalb des Setzrohrs in eine vorbestimmte Position; und
(iv) Verlagern des Setzrohrs relativ zu dem Kolbenstopfen mittels manueller Betätigung eines Betätigungsabschnitts einer Verlagerungseinrichtung.

Bereits an dieser Stelle sei darauf hingewiesen, dass sämtliche in Bezug auf die erfindungsgemäße Vorrichtung erwähnten Vorteile, Merkmale und Effekte auch auf das erfindungsgemäße Verfahren anwendbar sind und umgekehrt.

So erlaubt auch das erfindungsgemäße Verfahren, Kleinstserien von mit einem Arzneimittel befüllten Spritzen (und anderen spritzenähnlichen zylindrischen Röhren wie z.B. Karpulen, Doppelkammerkarpulen und Doppelkammerspritzen) ab einer Stückzahl von 1 bei gleichzeitiger Einhaltung von Serienbedingungen herstellen zu können.

Dabei kann der Kolbenstopfen in der vorbestimmten Position innerhalb des Spritzenzylinders angeordnet sein. Das heißt, der Kolbenstopfen kann in der vorbestimmten Position sowohl innerhalb des Setzrohrs als auch innerhalb des Spritzenzylinders angeordnet sein.

Nachdem der Kolbenstopfen die vorbestimmte Position innerhalb des Setzrohrs und des Spritzenzylinders erreicht hat, kann das Setzrohr relativ zum Kolbenstopfen in eine weitere Position verlagert werden, wobei der Kolbenstopfen vollständig aus dem Setzrohr tritt und derart expandiert, dass er mit einer Innenfläche des Spritzenzylinders in Kontakt tritt. Auf diese Weise wirkt der Kolbenstopfen mit dem Spritzenzylinder in einer abdichtenden Weise zusammen.

Das erfindungsgemäße Verfahren kann ferner den Schritt Befüllen des Spritzenzylinders und gegebenenfalls Verschließen eines Spritzenkopfes, bevor der Spritzenzylinder in die Aufnahme eingeführt wird, umfassen. Dieser Schritt des Befüllens des Spritzenzylinders mit einem Arzneimittel kann an einem dafür eingerichteten Abschnitt der erfindungsgemäßen Vorrichtung oder auch an einem von der erfindungsgemäßen Vorrichtung getrennten Abschnitt durchgeführt werden. Eine Befüllung kann beispielsweise mittels Pipette oder halbautomatischer Befüllung erfolgen, vorteilhafterweise in steriler Umgebung, etwa in einem Isolator. Es ist erfindungsgemäß möglich, die gesamte benötigte Ausrüstung zum Befüllen von Spritzenzylindern einerseits und zum Stopfensetzen andererseits in einen Isolator einzubringen. Damit kann sowohl das Befüllen als auch das Stopfensetzen steril erfolgen.

Ferner kann das Verfahren den Schritt Anordnen des Setzrohrs in einer Haltevorrichtung, bevor der Kolbenstopfen in das Setzrohr eingeführt wird, umfassen. Auf diese Weise können mit der Haltevorrichtung verschiedene Setzrohre austauschbar verbunden werden.

Das Verfahren kann ferner den Schritt Verlagern der Aufnahme mit dem Spritzenzylinder in eine vorbestimmte Position umfassen, bevor der Kolbenstopfen innerhalb des Setzrohrs verlagert wird, wobei sich das dem Spritzenzylinder zugewandte Ende des Setzrohrs in der vorbestimmten Position des Spritzenzylinders zumindest teilweise innerhalb des Spritzenzylinders befindet. In einer derartigen Ausführungsform des erfindungsgemäßen Verfahrens kann die Haltevorrichtung mit dem Setzrohr, beispielsweise relativ zu einem Gestell, mit welchem die Haltevorrichtung verbunden ist, fixiert verbleiben, wohingegen der Spritzenzylinder über das Setzrohr in die vorbestimmte Position verlagert werden kann.

Die vorbestimmte Position der Aufnahme mit dem Spritzenzylinder kann durch Anordnen der Aufnahme an einem Tiefenanschlag, der in der Aufnahme angebracht ist, erreicht werden. Hierdurch kann verhindert werden, dass der Spritzenzylinder über die vorbestimmte Position hinaus relativ zu dem Setzrohr verlagert wird, wodurch wiederum verhindert werden kann, dass das Setzrohr in Kontakt mit dem Arzneimittel kommt.

Vor dem Verlagern des Kolbenstopfens innerhalb des Setzrohrs können die Aufnahme mit dem Spritzenzylinder und das Setzrohr so justiert werden, dass sie im Wesentlichen koaxial zueinander ausgerichtet sind. Dies erlaubt ein relatives Verlagern des Setzrohrs und des Spritzenzylinders zueinander, ohne dass sich diese berühren, wodurch beispielsweise eine Innenwand des Spritzenzylinders kontaminiert werden könnte.

Ferner kann das Verlagern des Kolbenstopfens innerhalb des Setzrohrs durch eine Verlagerungseinrichtung bewirkt werden, die dazu eingerichtet ist, den Kolbenstopfen relativ zu dem Setzrohr zu verlagern. Dabei kann eine Verlagerungsrichtung der Verlagerungseinrichtung im Wesentlichen parallel oder koaxial zu einer Längsmittelachse des Setzrohrs und/oder des Spritzenzylinders verlaufen.

Insbesondere kann das Verlagern des Kolbenstopfens innerhalb des Setzrohrs durch Ausüben, vorteilhafterweise manuelles Ausüben, einer Druckkraft auf einen Stößel bewirkt werden, der einen Teil der Verlagerungseinrichtung darstellt und der dazu eingerichtet ist, den Kolbenstopfen relativ zum Setzrohr zu verlagern. Wie bereits mit Bezug auf die erfindungsgemäße Vorrichtung beschrieben, kann der Stößel den Kolbenstopfen durch das Setzrohr insbesondere derart verlagern, dass der Kolbenstopfen eine gewünschte Orientierung relativ zu dem Setzrohr beibehält, das heißt ein ungewolltes Verkippen des Kolbenstopfens verhindert werden kann.

Dabei kann die vorbestimmte Position des Kolbenstopfens vorteilhafterweise durch Anordnen eines an dem Stößel angeordneten Tiefenanschlags an eine Stößelführung erreicht werden. Auch hier kann ein Tiefenanschlag verhindern, dass der Kolbenstopfen über die vorbestimmte Position hinaus verlagert wird.

Vorteilhafterweise kann das Verlagern des Setzrohrs relativ zum Kolbenstopfen mittels manuellen Ausübens einer in eine erste Richtung wirkenden Betätigungskraft, vorteilhafterweise mit einem Finger, auf ein erstes Ende einer Wippeinrichtung bewirkt werden, wodurch an einem zweiten Ende der Wippeinrichtung, das dem ersten Ende im Wesentlichen entgegengesetzt angeordnet ist und welches vorteilhafterweise das Setzrohr zumindest teilweise umschließt, eine in einer zweiten Richtung wirkende Hebelkraft auf das Setzrohr übertragen wird, wobei die erste der zweiten Richtung im Wesentlichen entgegengesetzt sein können. Hierdurch kann das Setzrohr aus seiner Haltevorrichtung derart heraus verlagert werden, dass es sich von dem Spritzenzylinder entfernt, wobei es den Kolbenstopfen freigibt, so dass dieser expandiert und in Kontakt mit dem Spritzenzylinder tritt. Spätestens nach dem Entfernen der fertigen Spritze kann das Setzrohr in seiner Haltevorrichtung, beispielsweise aufgrund der Schwerkraft oder aufgrund eines neuen Kolbenstopfens, welcher in das Setzrohr eingeführt wird, in seine ursprüngliche Position zurück verlagert werden.

Dabei kann das Verlagern des Setzrohrs relativ zum Kolbenstopfen mittels
(i) Halten des Kolbenstopfens in der vorbestimmten Position innerhalb des Spritzenzylinders mittels Ausüben einer Haltekraft auf den Kolbenstopfen, vorteilhafterweise über einen fixierbaren Stößel, der einen Teil der Verlagerungseinrichtung darstellt und der dazu eingerichtet ist, den Kolbenstopfen relativ zum Setzrohr zu verlagern und der vorteilhafterweise mit einem daran angeordneten Tiefenanschlag an einer Stößelführung anliegt, und
(ii) Ausüben einer Druckkraft, vorteilhafterweise mit einem Finger, auf ein erstes Ende eines länglichen plattenartigen Elements, wodurch an einem zweiten Ende, das dem ersten Ende entgegengesetzt angeordnet ist und das Setzrohr zumindest teilweise umschließt, eine Hebelkraft auf das Setzrohr übertragen wird, deren Richtung der Druckkraft im Wesentlichen entgegengesetzt ist,
bewirkt werden, sodass der Kolbenstopfen vollständig aus dem Setzrohr tritt und derart expandiert, dass er mit einer Innenfläche des Spritzenzylinders in Kontakt tritt. Wie bereits voranstehend erwähnt, kann so ein Kolbenstopfen in einem Spritzenzylinder nahe an einen Füllstand eines in dem Spritzenzylinder angeordneten Arzneimittels herangeführt werden, ohne dass dabei ein Überdruck in dem Spritzenzylinder entsteht, welcher zum Beispiel Arzneimittel aus dem Spritzenzylinder austreiben kann.

In einer vorteilhaften Ausführungsform des erfindungsgemäßen Verfahrens kann das Verfahren die folgenden Schritte umfassen:
(i) Einführen des Kolbenstopfens in ein röhrenförmiges Setzrohr;
(ii) Einführen eines vorteilhafterweise befüllten Spritzenzylinders in eine dazu eingerichtete Aufnahme umfassend eine erste Zentrieraufnahme, die dazu eingerichtet ist, eine zweite Zentrieraufnahme zu haltern, und die zweite Zentrieraufnahme, die dazu eingerichtet ist, den Spritzenzylinder zu haltern;
(iii) Verlagern der zweiten Zentrieraufnahme mit dem Spritzenzylinder in eine vorbestimmte Position innerhalb der ersten Zentrieraufnahme, wobei die vorbestimmte Position vorteilhafterweise durch Anordnen der zweiten Zentrieraufnahme an einem Tiefenanschlag, der in der ersten Zentrieraufnahme angebracht ist, erreicht wird;
(iv) Anordnen des Setzrohrs mit dem Kolbenstopfen in eine Haltevorrichtung;
(v) gegebenenfalls Justieren der Aufnahme mit dem Spritzenzylinder und des Setzrohrs, sodass sie im Wesentlichen koaxial zueinander ausgerichtet sind;
(vi) Lösen einer Sicherungseinrichtung, die dazu eingerichtet ist, eine unbeabsichtigte Verlagerung des Kolbenstopfens relativ zu dem Setzrohr zu verhindern;
(vii) Verlagern des Kolbenstopfens innerhalb des Setzrohrs durch Ausüben, vorteilhafterweise manuelles Ausüben, einer Druckkraft auf einen Stößel, der dazu eingerichtet ist, den Kolbenstopfen relativ zum Setzrohr zu verlagern, wobei der Kolbenstopfen, der am dem Stößel entgegengesetzten Ende des Kolbenstopfens angeordnet ist, mittels der auf den Stößel ausgeübten Druckkraft in eine vorbestimmte Position verlagert wird, wobei die vorbestimmte Position des Kolbenstopfens vorteilhafterweise durch Anordnen eines an dem Stößel angeordneten Tiefenanschlags an eine Stößelführung erreicht wird; und
(viii) Verlagern des Setzrohrs relativ zum Kolbenstopfen mittels Halten des Kolbenstopfens in der vorbestimmten Position innerhalb des Spritzenzylinders mittels Ausüben einer Haltekraft auf den Kolbenstopfen über den Stößel, welcher vorteilhafterweise mit dem Tiefenanschlag an der Stößelführung anliegt; und

Ausüben einer Druckkraft, vorteilhafterweise mit einem Finger, auf ein erstes Ende eines länglichen plattenartigen Elements, wodurch an einem zweiten Ende, das dem ersten Ende entgegengesetzt angeordnet ist und das Setzrohr zumindest teilweise umschließt, eine Hebelkraft auf das Setzrohr übertragen wird, deren Richtung der Druckkraft im Wesentlichen entgegengesetzt ist,
wobei der Kolbenstopfen vollständig aus dem Setzrohr tritt und derart expandiert, dass er mit einer Innenfläche des Spritzenzylinders in Kontakt tritt.

Mit Bezug auf die obige Beschreibung des erfindungsgemäßen Verfahrens und der erfindungsgemäßen Vorrichtung zu den einzelnen Merkmalen dieser Ausführungsform kann diese Ausführungsform eine besonders einfache Realisierung darstellen, um Kleinstserien von mit Arzneimittel befüllten Spritzen, auch von unterschiedlichen Spritzenformaten, unter Einhaltung von Serienbedingungen herzustellen.

Im Folgenden wird die vorliegende Erfindung anhand eines Ausführungsbeispiels mit Bezug auf die begleitenden Zeichnungen in größerem Detail beschrieben werden. Es stellt dar:

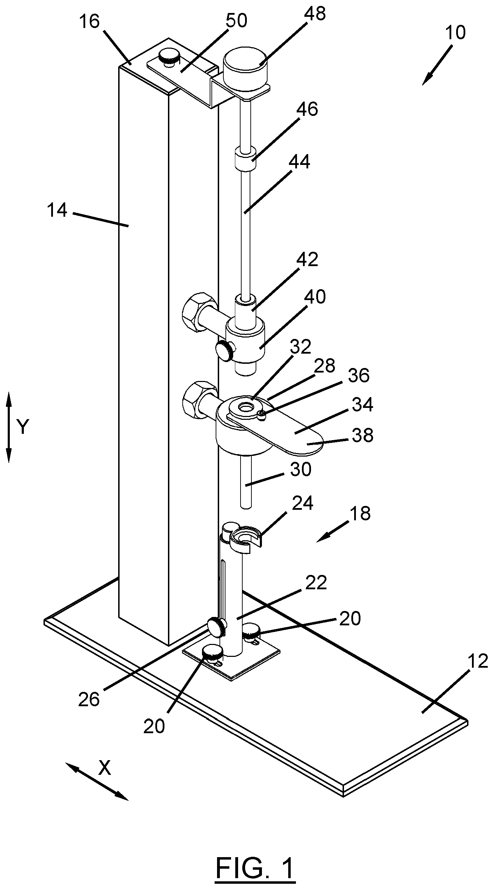

- Figur 1: eine perspektivische Ansicht einer Ausführungsform einer erfindungsgemäßen Vorrichtung.

In Figur 1 ist eine Ausführungsform der erfindungsgemäßen Vorrichtung allgemein mit dem Bezugszeichen 10 bezeichnet. Die Vorrichtung 10 umfasst eine Grundplatte 12 als eine Bodenplatte, auf welcher die Vorrichtung 10 aufsteht. Mit der Grundplatte 12 ist ein Stützelement 14 verbunden, welches hier als ein Vierkantrohr ausgebildet ist, welches an seinem der Grundplatte 12 entgegengesetzten Ende mit einem Deckel 16 verschweißt ist.

Mit der Grundplatte 12 ist ferner eine Spritzenaufnahme 18 in dem in Figur 1 dargestellten Ausführungsbeispiel über zwei Schrauben 20 verbunden. Die Verbindung der Spritzenaufnahme 18 mit der Grundplatte 12 erlaubt hier eine Verlagerung der Spritzenaufnahme 18 entlang einer Achse X auf das Stützelement 14 zu bzw. von diesem weg. Die Spritzenaufnahme 18 umfasst eine erste Zentrieraufnahme 22, welche dazu eingerichtet ist, eine zweite Zentrieraufnahme 24 aufzunehmen, wobei die zweite Zentrieraufnahme 24 wiederum dazu eingerichtet ist, einen nicht dargestellten Spritzenzylinder aufzunehmen. Die zweite Zentrieraufnahme 24 ist in der ersten Zentrieraufnahme 22 entlang einer Achse Y verlagerbar, wobei eine mit der zweiten Zentrieraufnahme 24 verbundene und mit der ersten Zentrieraufnahme 22 eingreifende Schraube 26 einen Tiefenanschlag 26 bildet und eine Justierung der beiden Zentrieraufnahmen 22, 24 zueinander in einer gewünschten Position ermöglicht.

Entlang der Richtung Y ist oberhalb der zweiten Zentrieraufnahme 24 eine Haltevorrichtung 28 mit dem Stützelement 14 derart verschraubt, dass die Haltevorrichtung 28 entlang der Achse X relativ zu dem Stützelement 14 verlagerbar ist, wenn die Schraubverbindung gelöst wird. Die Haltevorrichtung 28 weist eine zentrale Durchgangsbohrung auf, in welche ein Setzrohr 30 eingesetzt ist. Das Setzrohr 30 umfasst an seinem der zweiten Zentrieraufnahme 24 entgegengesetzten Ende des Röhrenabschnitts einen Kragen 32, welcher sich von dem Röhrenabschnitt des Setzrohrs 30 nach radial außen erstreckt. Der Kragen 32 definiert zum einen eine vorbestimmte Position des Setzrohrs 30 relativ zu der Haltevorrichtung 28. Zum anderen greift mit dem Kragen 32 ein Betätigungsabschnitt 34 ein, welcher das Setzrohr 30 zwischen Kragen 32 und Haltevorrichtung 28 zumindest teilweise umgibt.

Der Betätigungsabschnitt 34 ist hier als eine plattenartige Wippeinrichtung ausgebildet, welche sich hier über eine Stellschraube 36, über die eine Vorspannung bzw. eine Neigung des Betätigungsabschnitts 34 relativ zu der Haltevorrichtung 28 bzw. dem Setzrohr 30 eingestellt werden kann, an der Haltevorrichtung 28 abstützt, so dass eine Betätigung des Betätigungsabschnitts 34 an seinem dem Setzrohr 30 entgegengesetzten Ende 38, beispielsweise durch einen Fingerdruck eines Benutzers, in der Y-Richtung in Figur 1 nach unten ein Anheben des Setzrohrs 30 in der Y-Richtung in Figur 1 nach oben bewirkt.

In der Y-Richtung in Figur 1 oberhalb der Haltevorrichtung 28 ist mit dem Stützelement 14 eine Stößelführungsaufnahme 40 verbunden, welche ebenfalls relativ zu dem Stützelement 14 entlang der Achse X verlagerbar und justierbar ist. Mit der Stößelführungsaufnahme 40 ist eine Stößelführung 42 verbunden, in welcher ein Stößel 44 entlang der Y-Richtung verlagerbar angeordnet ist. Der dem Setzrohr 30 zugewandte Abschnitt des Stößels 44 weist einen Außendurchmesser auf, welcher kleiner ist als ein Innendurchmesser des Setzrohrs 30, so dass der Stößel 44 in das Setzrohr 30 eintauchen kann. An einer vorbestimmten, insbesondere einstellbaren, Position des Stößels 44 ist mit diesem ein Tiefenanschlag 46 verbunden, welcher derart mit der Stößelführung 42 in Kontakt treten kann, dass eine Verlagerung des Stößels in der Y-Richtung über eine durch den Tiefenanschlag 46 definierte Position hinaus verhindert wird.

An seinem dem Setzrohr 30 entgegengesetzten Ende weist der Stößel 44 einen Druckkopf 48 auf, welcher als Grifffläche für einen Bediener dient. In Figur 1 ist zu erkennen, dass mit dem Druckkopf 48 eine Sicherungseinrichtung 50 in Eingriff steht, welche eine Verlagerung des Stößels 44, insbesondere aus der in Figur 1 gezeigten Position entlang der Y-Richtung nach unten, verhindert. Die in Figur 1 dargestellte Ausführungsform der Sicherungseinrichtung 50 ist dazu eingerichtet, um die Achse Y verschwenkt zu werden, um eine Verlagerung des Stößels 44 freizugeben.

Im Folgenden sei das erfindungsgemäße Verfahren in einer möglichen Ausführungsform nochmals kurz anhand der erfindungsgemäßen Vorrichtung 10 beschrieben. Zunächst wird ein Kolbenstopfen zum Beispiel unter Verwendung einer Pinzette in das Setzrohr 30 eingelegt. Danach wird ein mit einem Arzneimittel befüllter Spritzenzylinder in die zweite Zentrieraufnahme 24 eingesetzt. Die zweite Zentrieraufnahme 24 wird nun in der Richtung Y in Figur 1 nach oben verlagert, bis eine weitere Verlagerung durch den Tiefenanschlag 26 begrenzt ist. In dieser Position wird die Schraube 26 angezogen, so dass die Relativposition der zweiten Zentrieraufnahme 24 zu der ersten Zentrieraufnahme 22 fixiert wird. In diesem Zustand ist das Setzrohr 30 in den Spritzenzylinder eingetaucht. Als Nächstes wird die Sicherungseinrichtung 50 zur Seite geschwenkt, so dass der Stößel 44 in der Y-Richtung nach unten verlagert werden kann bis der Tiefenanschlag 46 gegen die Stößelführung 42 anliegt. Während ein Bediener einen Druck auf den Druckkopf 48 aufrechterhält, drückt der Bediener auf das erste Ende 38 des Betätigungsabschnitts 34 in der Richtung Y von oben nach unten, wobei das Setzrohr 30 zumindest teilweise aus dem Spritzenzylinder nach oben verlagert wird. Dabei wird der Kolbenstopfen, welcher weiterhin durch den Stößel 44 in seiner Position relativ zu dem Spritzenzylinder gehalten wird, freigegeben, so dass dieser expandieren kann, um mit dem Spritzenzylinder in einer abdichtenden Weise einzugreifen.

Anschließend werden der Stößel 44 und die zweite Zentrieraufnahme 24 zurück in ihre Ausgangspositionen verlagert und der mit dem Kolbenstopfen versehene Spritzenzylinder kann aus der Vorrichtung 10 entnommen werden.

## Patentansprüche

1. Vorrichtung (10) zum manuellen Einführen eines Kolbenstopfens aus Elastomer, der einen Teil eines Kolbens bildet, in einen Spritzenzylinder, umfassend:
eine Aufnahme (18), die dazu eingerichtet ist, den Spritzenzylinder zu haltern, wobei die Aufnahme (18) eine erste Zentrieraufnahme (22), welche dazu eingerichtet ist, eine zweite Zentrieraufnahme (24) aufzunehmen, umfasst und die zweite Zentrieraufnahme (24) in der ersten Zentrieraufnahme (22) entlang einer Achse y verlagerbar ist;
ein röhrenförmiges Setzrohr (30), das dazu eingerichtet ist, in seinem Inneren den Kolbenstopfen aufzunehmen;
eine Haltevorrichtung (28), die dazu eingerichtet ist, das Setzrohr (30) relativ zu der Aufnahme (18) zu haltern; und
eine Verlagerungseinrichtung (40, 42, 44, 46, 48), die dazu eingerichtet ist, den Kolbenstopfen relativ zu dem Setzrohr (30) zu verlagern, wobei die Verlagerungseinrichtung eine Stößelführung (42), die dazu eingerichtet ist, einen Stößel (44) relativ zu dem Setzrohr (30) auszurichten, und
den Stößel (44), der dazu eingerichtet ist, den Kolbenstopfen relativ zum Setzrohr (30) zu verlagern,
umfasst, wobei der Stößel (44) einen zylindrischen Abschnitt an dem der Haltevorrichtung (28) zugewandten Ende aufweist, dessen Außendurchmesser kleiner ist als ein lichter Innendurchmesser des Setzrohrs (30) und wobei der Stößel (44) einen Tiefenanschlag (46) und nach außen erstreckenden Knopf (48) umfasst, der dazu eingerichtet ist, manuell betätigt zu werden, um den Kolbenstopfen relativ zu dem Setzrohr (30) zu verlagern.

2. Vorrichtung (10) nach Anspruch 1, **dadurch gekennzeichnet, dass** der Betätigungsabschnitt (34) der Haltevorrichtung (28) eine vorteilhafterweise als längliches plattenartiges Element ausgestaltete Wippeinrichtung (34) umfasst, die dazu eingerichtet ist, eine in einer ersten Richtung wirkende manuelle Betätigungskraft, die an einem ersten Ende der Wippeinrichtung (34) eingegeben worden ist, in eine in einer zweiten Richtung wirkende Hebelkraft an einem zweiten Ende der Wippeinrichtung (34), das dem ersten Ende entgegengesetzt angeordnet ist, zu übertragen, wobei die erste Richtung der zweiten Richtung im Wesentlichen entgegengesetzt ist.

3. Vorrichtung (10) nach einem der vorhergehenden Ansprüche, **dadurch gekennzeichnet, dass** die Haltevorrichtung (28) und/oder die Verlagerungseinrichtung (40, 42, 44, 46, 48) und/oder die Aufnahme (18) relativ zueinander justierbar sind, vorteilhafterweise über jeweils mindestens eine Schraubverbindung (26), und/oder
**dadurch gekennzeichnet, dass** das Setzrohr (30) ferner dazu eingerichtet ist, derart relativ zu der Aufnahme (18) verlagerbar zu sein, dass es in einer vorbestimmten Position seines Verlagerungsweges zumindest teilweise in einen Innenraum eines an der Aufnahme (18) angeordneten Spritzenzylinders hineinragt, und/oder
**dadurch gekennzeichnet, dass** das Setzrohr (30) an einem Ende einen sich radial nach außen erstreckenden Kragen (32) aufweist, und/oder **dadurch gekennzeichnet, dass** die zum Haltern des Spritzenzylinders eingerichtete Aufnahme (18) mindestens zwei Teile umfasst.

4. Vorrichtung (10) nach einem der vorhergehenden Ansprüche, **dadurch gekennzeichnet, dass** die zum Haltern des Setzrohrs (30) eingerichtete Haltevorrichtung (28) das Setzrohr (30) zumindest teilweise umschließt, wobei die Haltevorrichtung (28) vorteilhafterweise hohlzylinderförmig mit einer bevorzugt zur Mittelachse der Haltevorrichtung (28) konzentrischen Bohrung zur Aufnahme (18) des Setzrohrs (30) ausgestaltet ist.

5. Vorrichtung nach einen der vorhergehenden Ansprüche,
**dadurch gekennzeichnet, dass**
der Knopf (48) mit einem Finger betätigt wird und/oder der Knopf (48) an dem der Haltevorrichtung entgegengesetzten Ende angeordnet ist.

6. Vorrichtung nach einem der vorangehenden Ansprüche,
**dadurch gekennzeichnet, dass**
der Stößel (44) einen justierbaren Tiefenanschlag umfasst, der dazu eingerichtet ist, dass Ende des Verlagerungsweges des Kolbenstopfens in dem Setzrohr durch die Verlagerungseinrichtung festzulegen.

7. Vorrichtung nach einem der vorangegangenen Ansprüche,
**dadurch gekennzeichnet, dass**
die Verlagerungseinrichtung eine Stößelführung umfasst, die dazu eingerichtet ist, einen Stößel (44) relativ zu dem Setzrohr auszurichten.

8. Vorrichtung nach Anspruch 7,
**dadurch gekennzeichnet, dass**
der Stößel dazu eingerichtet ist, den Kolbenstopfen relativ zum Setzrohr zu verlagern.

9. Vorrichtung nach einem der vorangegangenen Ansprüche,
**dadurch gekennzeichnet, dass**
die Verlagerungseinrichtung ferner eine Stößelführungsaufnahme umfasst, die dazu eingerichtet ist, die Stößelführung zu halten.

10. Vorrichtung nach einem der vorangegangenen Ansprüche,
**dadurch gekennzeichnet, dass**
der Stößel einen zylindrischen Abschnitt an dem der Haltevorrichtung zugewandten Ende aufweist, dessen Außendurchmesser kleiner ist als ein lichter Innendurchmesser des Setzrohres.

11. Vorrichtung (10) nach einem der vorhergehenden Ansprüche, umfassend:
eine Grundplatte (12);
ein Stützelement (14), das mit der Grundplatte (12) verbunden ist;
eine vorteilhafterweise an der Grundplatte (12) angebrachte Aufnahme (18) umfassend
eine erste Zentrieraufnahme (22), die dazu eingerichtet ist, eine zweite Zentrieraufnahme (24) zu haltern und die vorteilhafterweise einen Tiefenanschlag (26) aufweist, und
die zweite Zentrieraufnahme (24), die dazu eingerichtet ist, den Spritzenzylinder zu haltern;
ein röhrenförmiges Setzrohr (30), das dazu eingerichtet ist, in seinem Inneren den Kolbenstopfen aufzunehmen;
eine vorteilhafterweise an dem Stützelement (14) angebrachte Haltevorrichtung (28), die dazu eingerichtet ist, das Setzrohr (30) relativ zu der Aufnahme (18) zu haltern und die vorteilhafterweise das Setzrohr (30) zumindest teilweise umschließt, wobei die Haltevorrichtung (28) eine als längliches plattenartiges Element ausgestaltete Wippeinrichtung (34) umfasst, die dazu eingerichtet ist, das Setzrohr (30) relativ zu dem Kolbenstopfen zu verlagern, wobei die Wippeinrichtung (34) vorteilhafterweise ein erstes Ende (38), das dazu eingerichtet ist, mit einem Finger betätigt zu werden, und ein zweites Ende, das dem ersten Ende (38) im Wesentlichen entgegengesetzt angeordnet ist und welches das Setzrohr (30) zumindest teilweise umschließt, aufweist, und
eine Verlagerungseinrichtung (40, 42, 44, 46, 48), umfassend eine Stößelführung (42), die dazu eingerichtet ist, einen Stößel (44) relativ zu dem Setzrohr (30) auszurichten, wobei die Stößelführung (42) vorteilhafterweise direkt oder indirekt justierbar ist;
den Stößel (44), der dazu eingerichtet ist, den Kolbenstopfen relativ zum Setzrohr (30) zu verlagern und der vorteilhafterweise einen Tiefenanschlag (46) aufweist, und
eine vorteilhafterweise an dem Stützelement (14) angebrachte Stößelführungsaufnahme (40), die dazu eingerichtet ist, die Stößelführung (42) zu haltern; und gegebenenfalls
eine Sicherungseinrichtung (50), die dazu eingerichtet ist, eine unbeabsichtigte Verlagerung des Kolbenstopfens relativ zu dem Setzrohr (30) zu verhindern, wobei die Sicherungseinrichtung (50) vorteilhafterweise schwenkbar mit dem mindestens einen Stützelement (14) verbunden ist.

12. Verfahren zum manuellen flüssigkeitsdichten Einführen eines Kolbenstopfens aus Elastomer, der einen Teil eines Kolbens bildet, in einen Spritzenzylinder, umfassend die Schritte:
(i) Einführen des Kolbenstopfens in ein röhrenförmiges Setzrohr (30);
(ii) Einführen des Spritzenzylinders in eine zweite Zentrieraufnahme (24) einer dazu eingerichteten Aufnahme (18);
(iii) Verlagern des Kolbenstopfens innerhalb des Setzrohrs (30) in eine vorbestimmte Position durch eine Verlagerungseinrichtung und
(iv) Verlagern des Setzrohrs (30) relativ zu dem Kolbenstopfen mittels manueller Betätigung eines Betätigungsabschnitts (34) einer Verlagerungseinrichtung (40, 42, 44, 46, 48), derart, dass das Setzrohr (30) sich vom Spritzenzylinder entfernt, wobei das Setzrohr den Kolbenstopfen freigibt, so dass dieser expandiert und in Kontakt mit dem Spritzenzylinder tritt.

13. Verfahren nach Anspruch 12, **dadurch gekennzeichnet, dass** das Verlagern des Kolbenstopfens innerhalb des Setzrohrs (30) durch eine Verlagerungseinrichtung (40, 42, 44, 46, 48) bewirkt wird, die dazu eingerichtet ist, den Kolbenstopfen relativ zu dem Setzrohr (30) zu verlagern.

14. Verfahren nach Anspruch 12 oder 13, **dadurch gekennzeichnet, dass** das Verlagern des Setzrohrs (30) relativ zum Kolbenstopfen mittels manuellen Ausübens einer in eine erste Richtung wirkenden Betätigungskraft, vorteilhafterweise mit einem Finger, auf ein erstes Ende (38) einer Wippeinrichtung (34) bewirkt wird, wodurch an einem zweiten Ende der Wippeinrichtung (34), das dem ersten Ende (38) im Wesentlichen entgegengesetzt angeordnet ist und welches vorteilhafterweise das Setzrohr (30) zumindest teilweise umschließt, eine in einer zweiten Richtung wirkende Hebelkraft auf das Setzrohr (30) übertragen wird, wobei die erste der zweiten Richtung im Wesentlichen entgegengesetzt ist, wobei das Verlagern des Setzrohres relativ zum Kolbenstreifen mittels
(i) Halten des Kolbenstopfens in der vorbestimmten Position innerhalb des Spritzenzylinders mittels Ausüben einer Haltekraft auf den Kolbenstopfen, vorteilhafterweise über einen fixierbaren Stößel (44), der einen Teil der Verlagerungseinrichtung (40, 42, 44, 46, 48) darstellt und der dazu eingerichtet ist, den Kolbenstopfen relativ zum Setzrohr (30) zu verlagern und der vorteilhafterweise mit einem daran angeordneten Tiefenanschlag (46) an einer Stößelführung (42) anliegt, und
(ii) Ausüben einer Druckkraft, vorteilhafterweise mit einem Finger, auf ein erstes Ende (38) eines länglichen plattenartigen Elements (34), wodurch an einem zweiten Ende, das dem ersten Ende (38) entgegengesetzt angeordnet ist und das Setzrohr (30) zumindest teilweise umschließt, eine Hebelkraft auf das Setzrohr (30) übertragen wird, deren Richtung der Druckkraft im Wesentlichen entgegengesetzt ist,
bewirkt wird, sodass der Kolbenstopfen vollständig aus dem Setzrohr (30) tritt und derart expandiert, dass er mit einer Innenfläche des Spritzenzylinders in Kontakt tritt.

15. Verfahren nach einem der Ansprüche 12-14, **dadurch gekennzeichnet, dass** das Verfahren die folgenden Schritte umfasst:
(i) Einführen des Kolbenstopfens in ein röhrenförmiges Setzrohr (30);
(ii) Einführen eines vorteilhafterweise befüllten Spritzenzylinders in eine dazu eingerichtete Aufnahme (18) umfassend eine erste Zentrieraufnahme (22), die dazu eingerichtet ist, eine zweite Zentrieraufnahme (24) zu haltern, und die zweite Zentrieraufnahme (24), die dazu eingerichtet ist, den Spritzenzylinder zu haltern;(iii) Verlagern der zweiten Zentrieraufnahme (24) mit dem Spritzenzylinder in eine vorbestimmte Position innerhalb der ersten Zentrieraufnahme (22), wobei die vorbestimmte Position vorteilhafterweise durch Anordnen der zweiten Zentrieraufnahme (24) an einem Tiefenanschlag (26), der in der ersten Zentrieraufnahme (22) angebracht ist, erreicht wird;
(iv) Anordnen des Setzrohrs (30) mit dem Kolbenstopfen in eine Haltevorrichtung (28);
(v) gegebenenfalls Justieren der Aufnahme (18) mit dem Spritzenzylinder und des Setzrohrs (30), sodass sie im Wesentlichen koaxial zueinander ausgerichtet sind;
(vi) Lösen einer Sicherungseinrichtung (50), die dazu eingerichtet ist, eine unbeabsichtigte Verlagerung des Kolbenstopfens relativ zu dem Setzrohr (30) zu verhindern;
(vii) Verlagern des Kolbenstopfens innerhalb des Setzrohrs (30) durch Ausüben, vorteilhafterweise manuelles Ausüben, einer Druckkraft auf einen Stößel (44), der dazu eingerichtet ist, den Kolbenstopfen relativ zum Setzrohr (30) zu verlagern, wobei der Kolbenstopfen, der am dem Stößel (44) entgegengesetzten Ende des Kolbenstopfens angeordnet ist, mittels der auf den Stößel (44) ausgeübten Druckkraft in eine vorbestimmte Position verlagert wird, wobei die vorbestimmte Position des Kolbenstopfens vorteilhafterweise durch Anordnen eines an dem Stößel (44) angeordneten Tiefenanschlags (46) an eine Stößelführung (42) erreicht wird; und
(viii) Verlagern des Setzrohrs (30) relativ zum Kolbenstopfen mittels Halten des Kolbenstopfens in der vorbestimmten Position innerhalb des Spritzenzylinders mittels Ausüben einer Haltekraft auf den Kolbenstopfen über den Stößel (44), welcher vorteilhafterweise mit dem Tiefenanschlag (46) an der Stößelführung (42) anliegt; und Ausüben einer Druckkraft, vorteilhafterweise mit einem Finger, auf ein erstes Ende (38) eines länglichen plattenartigen Elements (34), wodurch an einem zweiten Ende, das dem ersten Ende (38) entgegengesetzt angeordnet ist und das Setzrohr (30) zumindest teilweise umschließt, eine Hebelkraft auf das Setzrohr (30) übertragen wird, deren Richtung der Druckkraft im Wesentlichen entgegengesetzt ist,
wobei der Kolbenstopfen vollständig aus dem Setzrohr (30) tritt und derart expandiert, dass er mit einer Innenfläche des Spritzenzylinders in Kontakt tritt.

## Claims

1. Device (10) for manually inserting an elastomer plunger plug, which forms part of a plunger, into a syringe cylinder, comprising:
a receptacle (18) designed to hold the syringe cylinder, wherein the receptacle (18) comprises a first centering receptacle (22) designed to receive a second centering receptacle (24), and the second centering receptacle (24) is displaceable in the first centering receptacle (22) along an axis y;
a tubular setting tube (30) adapted to receive the plunger plug therein;
a holding device (28) adapted to hold the setting tube (30) relative to the receptacle (18);
and a displacement device (40, 42, 44, 46, 48) adapted to displace the plunger plug relative to the setting tube (30), wherein the displacement device comprises a plunger guide (42) adapted to align a plunger (44) relative to the setting tube (30), and
the plunger (44) adapted to displace the plunger plug relative to the setting tube (30),
wherein the plunger (44) has a cylindrical section at the end facing the holding device (28) whose outer diameter is smaller than an inside diameter of the setting tube (30), and wherein the plunger (44) comprises a depth stop (46) and an outwardly extending button (48) adapted to be manually actuated to displace the plunger plug relative to the setting tube (30).

2. Device (10) according to claim 1, **characterized in that** the actuating section (34) of the holding device (28) comprises a rocker device (34), advantageously designed as an elongated plate-like element, which is arranged to convert a manual operating force acting in a first direction, which has been applied to a first end of the rocker device (34), into a lever force acting in a second direction at a second end of the rocker device (34), which is arranged opposite the first end, wherein the first direction is substantially opposite to the second direction.

3. Device (10) according to one of the preceding claims, **characterized in that** the holding device (28) and/or the displacement device (40, 42, 44, 46, 48) and/or the receptacle (18) are adjustable relative to each other, advantageously via at least one screw connection (26) in each case, and/or **characterized in that** the setting tube (30) is further designed to be displaceable relative to the receptacle (18) in such a way that, in a predetermined position of its displacement path, it protrudes at least partially into an interior space of an syringe cylinder arranged on the receptacle (18), and/or
**characterized in that** the setting tube (30) has a collar (32) extending radially outward at one end, and/or
**characterized in that** the receptacle (18) designed to hold the syringe cylinder comprises at least two parts.

4. Device (10) according to one of the preceding claims, **characterized in that** the holding device (28) designed to hold the setting tube (30) surrounds the setting tube (30) at least partially, wherein the holding device (28) is advantageously designed to be hollow cylindrical with a bore preferably concentric to the central axis of the holding device (28) for receiving (18) the setting tube (30).

5. Device according to one of the preceding claims,
**characterized in that**
the button (48) is actuated with a finger and/or the button (48) is arranged at the end opposite the holding device.

6. Device according to one of the preceding claims,
**characterized in that**
the plunger (44) comprises an adjustable depth stop which is designed to determine the end of the displacement path of the plunger plug in the setting tube by means of the displacement device.

7. Device according to one of the preceding claims,
**characterized in that**
the displacement device comprises a plunger guide which is designed to align a plunger (44) relative to the setting tube.

8. Device according to claim 7,
**characterized in that**
the plunger is designed to displace the plunger plug relative to the setting tube.

9. Device according to one of the preceding claims,
**characterized in that**
the displacement device further comprises a plunger guide receptacle designed to hold the plunger guide.

10. Device according to one of the preceding claims,
**characterized in that**
the plunger has a cylindrical section at the end facing the holding device, the outer diameter of which is smaller than the inside diameter of the setting tube.

11. Device (10) according to one of the preceding claims, comprising:
a base plate (12);
a support element (14) connected to the base plate (12);
a receptacle (18) advantageously mounted on the base plate (12) comprising a first centering receptacle (22) adapted to hold a second centering receptacle (24) and advantageously having a depth stop (26), and
the second centering receptacle (24), which is designed to hold the syringe cylinder;
a tubular setting tube (30), which is designed to accommodate the plunger plug inside it;
a holding device (28) advantageously attached to the support element (14), which is designed to hold the setting tube (30) relative to the receptacle (18) and which advantageously at least partially encloses the setting tube (30), wherein the holding device (28) comprises a rocker device (34) designed as an elongated plate-like element, which is designed to displace the setting tube (30) relative to the plunger plug, wherein the rocker device (34) advantageously has a first end (38) that is designed to be actuated by a finger and a second end that is arranged substantially opposite the first end (38) and which at least partially encloses the setting tube (30), and
a displacement device (40, 42, 44, 46, 48) comprising a plunger guide (42) adapted to align a plunger (44) relative to the setting tube (30), wherein the plunger guide (42) is advantageously directly or
indirectly adjustable;
the plunger (44) which is adapted to displace the plunger plug relative to the setting tube (30) and advantageously comprises a depth stop (46), and
a plunger guide receptacle (40) advantageously attached to the support element (14), which is designed to hold the plunger guide (42); and, optionally,
a locking device (50) designed to prevent unintentional displacement of the plunger plug relative to the setting tube (30), wherein the locking device (50) is advantageously pivotably connected to the at least one support element (14).

12. Method for manually inserting an elastomeric plunger plug, which forms part of a plunger, into a syringe cylinder in a liquid-tight manner, comprising the steps of:
(i) inserting the plunger plug into a tubular setting tube (30);
(ii) inserting the syringe cylinder into a second centering receptacle (24) of a receptacle (18) designed for this purpose;
(iii) displacing the plunger plug within the setting tube (30) to a predetermined position by means of a displacement device; and
(iv) displacing the setting tube (30) relative to the plunger plug by manually actuating an actuating section (34) of a displacement device (40, 42, 44, 46, 48) such that the setting tube (30) moves away from the syringe cylinder, wherein the setting tube releases the plunger plug so that it expands and comes into contact with the syringe cylinder.

13. Method according to claim 12, **characterized in that** the displacement of the plunger plug within the setting tube (30) is effected by a displacement device (40, 42, 44, 46, 48) which is designed to displace the plunger plug relative to the setting tube (30).

14. Method according to claim 12 or 13, **characterized in that** the displacement of the setting tube (30) relative to the plunger plug is effected by manually exerting an actuating force acting in a first direction, advantageously with a finger, on a first end (38) of a rocker device (34), whereby at a second end of the rocker device (34) which is arranged substantially opposite the first end (38) and which advantageously at least partially encloses the setting tube (30), a lever force acting in a second direction is transmitted to the setting tube (30), wherein the first direction is substantially opposite to the second direction, whereby the displacement of the setting tube relative to the plunger is made by means of
(i) holding the plunger plug in the predetermined position within the syringe cylinder by exerting a holding force on the plunger plug, advantageously via a fixable plunger (44) which forms part of the displacement device (40, 42, 44, 46, 48) and which is adapted to displace the plunger plug relative to the setting tube (30) and which advantageously abuts against a plunger guide (42) with a depth stop (46) arranged thereon, and
(ii) exerting a pressure force, advantageously with a finger, to a first end (38) of an elongated plate-like element (34), thereby exerting a lever force on the setting tube (30) at a second end which is arranged opposite the first end (38) and at least partially encloses the setting tube (30), the direction of which is substantially opposite to that of the pressure force,
causing the plunger plug to emerge completely from the setting tube (30) and expand so that it comes into contact with an inner surface of the syringe cylinder.

15. Method according to one of claims 12-14, **characterized in that** the method comprises the following steps of:
(i) inserting the plunger plug into a tubular setting tube (30);
(ii) inserting a syringe cylinder, preferably filled, into a receptacle (18) designed for this purpose, comprising a first centering receptacle (22) designed to hold a second centering receptacle (24), and the second centering receptacle (24) designed to hold the syringe cylinder;
(iii) moving the second centering receptacle (24) with the syringe cylinder into a predetermined position within the first centering receptacle (22), wherein the predetermined position is advantageously reached by arranging the second centering receptacle (24) at a depth stop (26) mounted in the first centering receptacle (22);
(iv) arranging the setting tube (30) with the plunger plug in a holding device (28);
(v) optionally, adjusting the receptacle (18) with the syringe cylinder and the setting tube (30) so that they are aligned substantially coaxially with each other;
(vi) releasing a locking device (50) which is designed to prevent unintentional displacement of the plunger plug relative to the setting tube (30);
(vii) displacing the plunger plug within the setting tube (30) by exerting, preferably manually, a pressure force on a plunger (44) that is designed to displace the plunger plug relative to the setting tube (30), wherein the plunger plug, which is arranged at the end of the plunger plug opposite the plunger (44), is displaced into a predetermined position by means of the pressure force exerted on the plunger (44) to a predetermined position, wherein the predetermined position of the plunger plug is advantageously achieved by arranging a depth stop (46) arranged on the plunger (44) on a plunger guide (42); and
(viii) displacing the setting tube (30) relative to the plunger plug by holding the plunger plug in the predetermined position within the syringe cylinder by exerting a holding force on the plunger plug via the plunger (44), which is advantageously in contact with the depth stop (46) on the plunger guide (42); and
exerting a pressure force, advantageously with a finger, to a first end (38) of an elongated plate-like element (34), thereby transmitting a lever force to the setting tube (30) at a second end opposite the first end (38) and at least partially enclosing the setting tube (30) whose direction is substantially opposite to that of the pressure force, wherein the plunger plug emerges completely from the setting tube (30) and expands so that it comes into contact with an inner surface of the syringe cylinder.

## Revendications

1. Dispositif (10) destiné à l'introduction manuelle d'un bouchon de piston en élastomère, qui forme une partie d'un piston, dans un cylindre de seringue, comprenant :
un réceptacle (18) qui est conçu pour maintenir le cylindre de seringue, le réceptacle (18) comprenant un premier réceptacle de centrage (22) qui est conçu pour recevoir un deuxième réceptacle de centrage (24), et le deuxième réceptacle de centrage (24) pouvant être déplacé dans le premier réceptacle de centrage (22) le long d'un axe y ;
un tube de pose (30) de forme tubulaire qui est conçu pour recevoir intérieurement le bouchon de piston ;
un dispositif de maintien (28) qui est conçu pour maintenir le tube de pose (30) relativement au réceptacle (18) ; et
un équipement de déplacement (40, 42, 44, 46, 48) qui est conçu pour déplacer le bouchon de piston relativement au tube de pose (30), l'équipement de déplacement comprenant un guide de poussoir (42) qui est conçu pour aligner un poussoir (44) relativement au tube de pose (30), et
le poussoir (44) qui est conçu pour déplacer le bouchon de piston relativement au tube de pose (30),
le poussoir (44) présentant un tronçon cylindrique à l'extrémité tournée vers le dispositif de maintien (28) dont le diamètre extérieur est plus petit qu'un diamètre interne libre du tube de pose (30), et le poussoir (44) comprenant une butée de profondeur (46) et un bouton (48) s'étendant vers l'extérieur qui est conçu pour être actionné manuellement afin de déplacer le bouchon de piston relativement au tube de pose (30).

2. Dispositif (10) selon la revendication 1, **caractérisé en ce que** le tronçon d'actionnement (34) du dispositif de maintien (28) comprend un équipement de bascule (34), constitué avantageusement comme un élément oblong de type plaque, qui est conçu pour convertir une force d'actionnement manuelle, agissant dans une première direction, qui a été entrée à une première extrémité de l'équipement de bascule (34), en une force de levier agissant dans une deuxième direction à une deuxième extrémité de l'équipement de bascule (34) qui est opposée à la première extrémité, la première direction étant essentiellement opposée à la deuxième direction.

3. Dispositif (10) selon l'une des revendications précédentes, **caractérisé en ce que** le dispositif de maintien (28) et/ou l'équipement de déplacement (40, 42, 44, 46, 48) et/ou le réceptacle (18) peuvent être ajustés les uns par rapport aux autres, avantageusement via respectivement au moins un assemblage vissé (26), et/ou
**caractérisé en ce que** le tube de pose (30) est en outre conçu pour pouvoir être déplacé relativement au réceptacle (18) de telle manière que, dans une position prédéfinie de sa course de déplacement, il dépasse intérieurement au moins partiellement dans un espace intérieur d'un cylindre de seringue disposé sur le réceptacle (18), et/ou
**caractérisé en ce que**, à une extrémité, le tube de pose (30) présente une collerette (32) s'étendant radialement vers l'extérieur, et/ou
**caractérisé en ce que** le réceptacle (18) conçu pour maintenir le cylindre de seringue comprend au moins deux parties.

4. Dispositif (10) selon l'une des revendications précédentes, **caractérisé en ce que** le dispositif de maintien (28) conçu pour maintenir le tube de pose (30) entoure au moins partiellement le tube de pose (30), le dispositif de maintien (28) étant avantageusement constitué en forme de cylindre creux avec un alésage, de préférence concentrique à l'axe central du dispositif de maintien (28), vers le réceptacle (18) du tube de pose (30).

5. Dispositif selon l'une des revendications précédentes,
**caractérisé en ce que**
le bouton (48) est actionné avec un doigt et/ou le bouton (48) est disposé à l'extrémité opposée au dispositif de maintien.

6. Dispositif selon l'une des revendications précédentes,
**caractérisé en ce que**
le poussoir (44) comprend une butée de profondeur ajustable qui est conçue pour fixer l'extrémité de la course de déplacement du bouchon de piston dans le tube de pose par l'équipement de déplacement.

7. Dispositif selon l'une des revendications précédentes,
**caractérisé en ce que**
l'équipement de déplacement comprend un guide de poussoir qui est conçu pour aligner un poussoir (44) relativement au tube de pose.

8. Dispositif selon la revendication 7,
**caractérisé en ce que**
le poussoir est conçu pour aligner le bouchon de piston relativement au tube de pose.

9. Dispositif selon l'une des revendications précédentes,
**caractérisé en ce que**
l'équipement de déplacement comprend en outre un réceptacle de guide de poussoir qui est conçu pour maintenir le guide de poussoir.

10. Dispositif selon l'une des revendications précédentes,
**caractérisé en ce que**
le poussoir présente un tronçon cylindrique à l'extrémité tournée vers le dispositif de maintien dont le diamètre extérieur est plus petit qu'un diamètre intérieur libre du tube de pose.

11. Dispositif (10) selon l'une des revendications précédentes,
comprenant
une plaque de base (12) ;
un élément de support (14) qui est raccordé à la plaque de base (12) ;
un réceptacle (18), avantageusement mis en place sur la plaque de base (12), comprenant
un premier réceptacle de centrage (22) qui est conçu pour maintenir un deuxième réceptacle de centrage (24) et qui présente avantageusement une butée de profondeur (26), et
le deuxième réceptacle de centrage (24) qui est conçu pour maintenir le cylindre de seringue ;
un tube de pose (30) de forme tubulaire qui est conçu pour recevoir intérieurement le bouchon de piston ;
un dispositif de maintien (28), avantageusement mis en place sur l'élément de support (14), qui est conçu pour maintenir le tube de pose (30) relativement au réceptacle (18) et qui entoure avantageusement au moins partiellement le tube de pose (30), le dispositif de maintien (28) comprenant un équipement de bascule (34), constitué comme un élément oblong de type plaque, qui est conçu pour déplacer le tube de pose (30) relativement au bouchon de piston, l'équipement de bascule (34) présentant avantageusement une première extrémité (38) qui est conçue pour être actionnée avec un doigt, et une deuxième extrémité qui est essentiellement opposée à la première extrémité (38) et qui entoure au moins partiellement le tube de pose (30), et
un équipement de déplacement (40, 42, 44, 46, 48), comprenant
un guide de poussoir (42) qui est conçu pour aligner un poussoir (44) relativement au tube de pose (30), le guide de poussoir (42) étant avantageusement ajustable directement ou indirectement ;
le poussoir (44) qui est conçu pour déplacer le bouchon de piston relativement au tube de pose (30) et qui présente avantageusement une butée de profondeur (46), et
un réceptacle de guide de poussoir (40), mis en place avantageusement sur l'élément de support (14), qui est conçu pour maintenir le guide de poussoir (42) ; et éventuellement
un équipement de blocage (50) qui est conçu pour empêcher un déplacement inopiné du bouchon de piston relativement au tube de pose (30), l'équipement de blocage (50) étant raccordé avantageusement de façon pivotante à l'au moins un élément de support (14).

12. Procédé d'introduction manuelle, de façon étanche aux liquides, d'un bouchon de piston en élastomère, qui forme une partie d'un piston, dans un cylindre de seringue, comprenant les étapes :
(i) introduction du bouchon de piston dans un tube de pose (30) de forme tubulaire ;
(ii) introduction du cylindre de seringue dans un deuxième réceptacle de centrage (24) d'un réceptacle (18) conçu à cet effet ;
(iii) déplacement du bouchon de piston à l'intérieur du tube de pose (30) dans une position prédéfinie par un équipement de déplacement, et
(iv) déplacement du tube de pose (30) relativement au bouchon de piston au moyen de l'actionnement manuel d'un tronçon d'actionnement (34) d'un équipement de déplacement (40, 42, 44, 46, 48) de telle manière que le tube de pose (30) s'éloigne du cylindre de seringue, le tube de pose libérant le bouchon de piston de telle manière que celui-ci se dilate et entre en contact avec le cylindre de seringue.

13. Procédé selon la revendication 12, **caractérisé en ce que** le déplacement du bouchon de piston à l'intérieur du tube de pose (30) est provoqué par un équipement de déplacement (40, 42, 44, 46, 48) qui est conçu pour déplacer le bouchon de piston relativement au tube de pose (30).

14. Procédé selon la revendication 12 ou 13, **caractérisé en ce que** le déplacement du tube de pose (30) relativement au bouchon de piston est provoqué au moyen de l'application manuelle d'une force d'actionnement agissant dans une première direction, avantageusement avec un doigt, sur une première extrémité (38) d'un équipement de bascule (34), grâce à quoi, à une deuxième extrémité de l'équipement de bascule (34) qui est disposée essentiellement à l'opposé de la première extrémité (38) et qui avantageusement entoure au moins partiellement le tube de pose (30), une force de levier agissant dans une deuxième direction est transmise au tube de pose (30), la première direction étant essentiellement opposée à la deuxième direction, en l'occurrence, le déplacement du tube de pose relativement au bouchon de piston peut étre provoqué au moyen de
(i) du maintien du bouchon de piston dans la position prédéfinie à l'intérieur du cylindre de seringue au moyen de l'application d'une force de maintien sur le bouchon de piston, avantageusement via un poussoir (44) pouvant être fixé qui représente une partie de l'équipement de déplacement (40, 42, 44, 46, 48) et qui est conçu pour déplacer le bouchon de piston relativement au tube de pose (30) et qui est avantageusement attenant à un guide de poussoir (42) par une butée de profondeur (46) qui est disposée dessus, et
(ii) de l'application d'une force de pression, avantageusement avec un doigt, à une première extrémité (38) d'un élément (34) oblong de type plaque, grâce à quoi, à une deuxième extrémité qui est disposée à l'opposé de la première extrémité (38) et qui entoure au moins partiellement le tube de pose (30), une force de levier est transmise au tube de pose (30), dont la direction est essentiellement opposée à la force de pression,
est provoqué, de sorte que le bouchon de piston sort complètement du tube de pose (30) et se dilate de telle manière qu'il entre en contact avec une surface intérieure du cylindre de seringue.

15. Procédé selon l'une des revendications 12-14 précédentes, **caractérisé en ce que** le procédé comprend les étapes suivantes :
(i) introduction du bouchon de piston dans un tube de pose (30) de forme tubulaire ;
(ii) introduction d'un cylindre de seringue avantageusement rempli dans un réceptacle (18) conçu à cet effet comprenant un premier réceptacle de centrage (22) qui est conçu pour maintenir un deuxième réceptacle de centrage (24), et le deuxième réceptacle de centrage (24) qui est conçu pour maintenir le cylindre de seringue ;
(iii) déplacement du deuxième réceptacle de centrage (24) avec le cylindre de seringue dans une position prédéfinie à l'intérieur du premier réceptacle de centrage (22), la position prédéfinie étant avantageusement atteinte par la disposition du deuxième réceptacle de centrage (24) sur une butée de profondeur (26) qui est mise en place dans le premier réceptacle de centrage (22) ;
(iv) disposition du tube de pose (30) avec le bouchon de piston dans un dispositif de maintien (28) ;
(v) éventuellement ajustement du réceptacle (18) avec le cylindre de seringue et du tube de pose (30) de telle manière qu'ils sont alignés essentiellement de façon coaxiale l'un avec l'autre ;
(vi) desserrage d'un équipement de blocage (50) qui est conçu pour empêcher un déplacement inopiné du bouchon de piston relativement au tube de pose (30) ;
(vii) déplacement du bouchon de piston à l'intérieur du tube de pose (30) par l'application, avantageusement par l'application manuelle, d'une force de pression sur un poussoir (44) qui est conçu pour déplacer le bouchon de piston relativement au tube de pose (30), le bouchon de piston, qui est disposé à l'extrémité du bouchon de piston opposée au poussoir (44), étant déplacé dans une position prédéfinie au moyen de la force de pression exercée sur le poussoir (44), la position prédéfinie du bouchon de piston étant atteinte avantageusement par la disposition sur un guide de poussoir (42) d'une butée de profondeur (46) disposée sur le poussoir (44) ; et
(viii) déplacement du tube de pose (30) relativement au bouchon de piston au moyen du maintien du bouchon de piston dans la position prédéfinie à l'intérieur du cylindre de seringue au moyen de l'application d'une force de maintien sur le bouchon de piston via le poussoir (44) qui est avantageusement attenant au guide de poussoir (42) par la butée de profondeur (46) ; et
application d'une force de pression, avantageusement avec un doigt, sur une première extrémité (38) d'un élément (34) oblong de type plaque, grâce à quoi, à une deuxième extrémité qui est opposée à la première extrémité (38) et qui entoure au moins partiellement le tube de pose (30), une force de maintien est transmise au tube de pose (30) dont la direction est essentiellement opposée à la force de pression,
le bouchon de piston sortant complètement du tube de pose (30) et se dilatant de telle manière qu'il entre en contact avec une surface intérieure du cylindre de seringue.
